(19) Europäisches Patentamt
European Patent Office
Office européen des brevets

(11) Veröffentlichungsnummer: **0 217 255 B1**

(12) **EUROPÄISCHE PATENTSCHRIFT**

(45) Veröffentlichungstag der Patentschrift: **12.06.91**

(51) Int. Cl.5: **G03C 7/32**, //C07D487/04, C07C317/00,(C07D487/04, 231:00,231:00)

(21) Anmeldenummer: **86113020.1**

(22) Anmeldetag: **22.09.86**

# (54) Farbfotografisches Aufzeichnungsmaterial mit leicht dispergierbaren Farbkupplern.

(30) Priorität: **03.10.85 DE 3535247**

(43) Veröffentlichungstag der Anmeldung:
**08.04.87 Patentblatt 87/15**

(45) Bekanntmachung des Hinweises auf die Patenterteilung:
**12.06.91 Patentblatt 91/24**

(84) Benannte Vertragsstaaten:
**BE DE FR GB**

(56) Entgegenhaltungen:
**EP-A- 0 073 636**
**CH-A- 472 056**

(73) Patentinhaber: **Agfa-Gevaert AG**
**W-5090 Leverkusen 1(DE)**

(72) Erfinder: **Wolff, Erich, Dr.**
**Balkhauser Weg 6**
**W-5650 Solingen(DE)**

EP 0 217 255 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein farbfotografisches Aufzeichnungsmaterial mit mindestens einer Silberhalogenidemulsionsschicht, das Kuppler enthält, die aufgrund der Anwesenheit spezieller Substituenten in organischen Lösungsmitteln hervorragend löslich sind.

Es ist bekannt, farbige fotografische Bilder durch chromogene Entwicklung herzustellen, d.h. dadurch, daß man bildmäßig belichtete Silberhalogenidemulsionsschichten in Gegenwart geeigneter Farbkuppler mittels geeigneter farbbildender Entwicklersubstanzen - sogenannter Farbentwickler - entwickelt, wobei das in Übereinstimmung mit dem Silberbild entstehende Oxidationsprodukt der Entwicklersubstanzen mit dem Farbkuppler unter Bildung eines Farbstoffbildes reagiert. Als Farbentwickler werden gewöhnlich aromatische, primäre Aminogruppen enthaltende Verbindungen, insbesondere solche vom p-Phenylendiamintyp, verwendet.

An die Farbkuppler sowie an die daraus durch chromogene Entwicklung erhaltenen Farbstoffe wird in der Praxis eine Reihe von Forderungen gestellt. So soll in erster Linie die Kupplungsgeschwindigkeit der Farbkuppler mit dem Oxidationsprodukt des Farbentwicklers möglichst groß sein. Die Farbkuppler sowie die daraus erhaltenen Farbstoffe müssen hinreichend stabil sein gegenüber Licht, erhöhter Temperatur und Feuchtigkeit. Dies gilt sowohl für die frisch hergestellten, als auch für die verarbeiteten Aufzeichnungsmaterialien. Beispielsweise darf der in den Bildweißen des verarbeiteten Materials noch vorhandene Restkuppler nicht vergilben. Außerdem sollen die Farbstoffe hinreichend beständig sein gegenüber gasförmigen reduzierenden oder oxidierenden Agentien. Sie müssen ferner diffusionsfest in der Bildschicht verankert sein und sollen sich bei der chromogenen Entwicklung als möglichst feines Korn abscheiden. Weiterhin dürfen die mechanischen Eigenschaften der Schichten durch die Farbkuppler nicht beeinträchtigt werden. Weiterhin müssen die aus den Farbkupplern bei der chromogenen Entwicklung entstehenden Farbstoffe eine günstige Absorptionskurve aufweisen mit einem Maximum, das der Farbe des jeweils gewünschten Teilbildes entspricht, und möglichst geringen Nebenarbsorptionen. So soll im Idealfall beispielsweise ein Purpur-farbstoff grünes Licht nahezu vollständig absorbieren und blaues sowie rotes Licht weitgehend durchlassen.

Weiterhin sollen die Farbkuppler in organischen Lösungsmitteln sehr gut löslich sein, so daß es möglich ist aus ihnen stabile Dispersionen in hydrophilen Medien, insbesondere den Bindemitteln der fotografischen Aufzeichnungsmaterialien herzustellen. Aus solchen Dispersionen wie auch aus den fertiggestellten farbfotografischen Aufzeichnungsmaterialien darf der Kuppler nicht agglomerieren oder auskristallisieren, was sich in einem Verlust an Empfindlichkeit und Farbausbeute äußern würde. Schließlich sind auch Kuppler erwünscht, die unempfindlich oder weniger empfindlich sind gegenüber Schwankungen in den Bedingungen der Verarbeitung, insbesondere Schwankungen der Temperatur, des pH-Wertes oder der Zusammensetzung der Verarbeitungsbäder.

Eine weitere wichtige Forderung ergibt sich daraus, daß moderne farbfotografische Aufzeichnungsmaterialien in zunehmendem Maße bei höheren Temperaturen verarbeitet werden. Dies macht wegen der damit verbundenen kürzeren Entwicklungszeiten vielfach die Anwendung lösungsvermittelnder Zusätze in den Verarbeitungsbädern erforderlich, um ausreichende Farbdichten erzielen zu können. Als lösungsvermittelnde Zusätze wurden beispielsweise hydrophile organische Lösungsmittel verwendet wie etwa Benzylalkohol, der ein gebräuchlicher Bestandteil in farbfotografischen Entwicklerbädern ist. Die Verwendung solcher lösungsvermittelnder Zusätze verursacht jedoch zusätzliche Probleme, die wenigsten teilweise darauf beruhen, daß diese Zusätze durch das zu verarbeitende farbfotografische Aufzeichnungsmaterial aus einem Behandlungsbad (z.B. Entwickler) in das nächste (z.B. Bleichfixierbad) verschleppt werden, wobei in ersterem eine Verarmung und im folgenden Bad eine Anreicherung stattfindet. Zur Erziehung gleichbleibender Ergebnisse ist daher eine ständige Überwachung und Auffrischung der Bäder erforderlich. Weiterhin sind aufwendige Vorkehrungen erforderlich um zu vermeiden, daß diese Zusätze in das Abwasser gelangen, wo sie den chemischen und biologischen Sauerstoffbedarf in unerwünschter Weise nachhaltig erhöhen. Es ist daher sehr erwünscht die Verwendung solcher lösungsvermittelnder Zusätze möglichst zu vermeiden.

In EP-A-0 073 636, US-A-4 503 141 und US-A-4 513 082 sind bereits Kuppler mit speziellen Ballastgruppen beschrieben, mit denen vorteilhafte Ergebnisse im Hinblick auf die oben erwähnten Ziele erreicht werden können. Die beschriebenen Kuppler sind jedoch nicht in jeder Hinsicht befriedigend und es besteht daher ein Bedarf an Kupplern mit weiter verbesserten Eigenschaften.

Kuppler der Formel

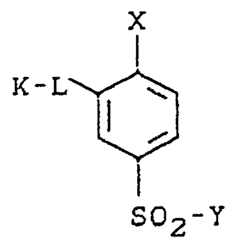

worin bedeuten

K einen (naphtholischen) Kupplerrest;

L -CONH-;

X Halogen, $-OR_1$, $-SR_1$, $-SO_2R_1$, -O-Aryl, -S-Aryl, $-SO_2$-Aryl oder $-NR_1R_2$;

$R_1$ Alkyl bis zu 18 C-Atomen oder Aralkyl;

$R_2$ H oder $R_1$ und

Y Alkyl, Aralkyl oder $-NR_1R_2$

sind beschrieben in CH-A-472 056.

Der Erfindung liegt die Aufgabe zugrunde ein farbfotografisches Aufzeichnungsmaterial mit Farbkupplern anzugeben, die eine hervorragende Löslichkeit in organischen Lösungsmitteln haben und daher leicht in die Schichten des farbfotografischen Aufzeichnungsmaterials eingearbeitet werden können. Das Aufzeichnungsmaterial soll für die Hochtemperaturverarbeitung geeignet sein und dabei auch in Abwesenheit lösungsvermittelnder organischer Zusätze wie Benzylalkohol Bildfarbstoffe mit hoher Farbdichte liefern.

Gegenstand der Erfindung ist ein farbfotografisches Aufzeichnungsmaterial mit mindestens einer Silberhalogenidemulsionsschicht und mindestens einem Kuppler, der einen mit einer Hydroxylgruppe und einer Sulfonylgruppe substituierten Phenylrest enthält, dadurch gekennzeichnet, daß der Kuppler der folgenden allgemeinen Formel I entspricht

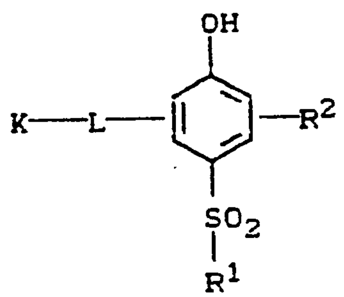

I

worin bedeuten

K einen Kupplerrest

L eine direkte Bindung oder ein Bindeglied

$R^1$ Alkyl, Aralkyl, Aryl oder Amino

$R^2$ Wasserstoff oder einen Rest -L-K.

Der in der Formel I dargestellte Kupplerrest enthält eine kuppelnde Funktion, d.h. eine Gruppe, die unter den Bedingungen der fotografischen Entwicklung zur Kupplung mit dem Oxidationsprodukt eines Farbentwicklers befähigt ist. Der Kupplerrest K stellt damit den Rest eines Kupplers dar, z.B. den Rest eines Gelbkupplers vom Typ des α-Acylacetanilids, eines Purpurkupplers vom Typ des 3-Anilino-oder 3-Acylamino-1-arylpyrazolons oder des Pyrazolotriazols, oder eines phenolischen oder naphtholischen Blaugrünkupplers. Bei den erfindungsgemäßen Kupplern kann es sich gleichermaßen um 2- oder 4-Äquivalentkuppler handeln, das bedeutet, die Kupplungsstelle ist entweder unsubstituiert oder substituiert mit einem Rest, der bei der Entwicklung freigesetzt werden kann. Bei solchen freisetzbaren Resten handelt es sich beispielsweise um Halogenatome oder um organische Reste, die über ein Sauerstoff-, Schwefel- oer Stickstoffatom mit der Kupplungsstelle verbunden sind. Vielfach sind solche Reste die Reste von heterocyclischen Verbindungen, die über ein Ringstickstoffatom mit der Kupplungsstelle verbunden sind.

Auch der in Formel I dargestellte durch eine Hydroxylgruppe und eine Sulfonylgruppe substituierte Phenylrest kann ein solcher abspaltbarer Rest sein, der gegebenenfalls über ein Bindeglied mit der Kupplungssstelle des Kupplers verbunden ist. In der Regel ist er aber über eine nicht kuppelnde Stelle mit

dem Kupplerrest K verknüpft.

Bei dem in Formel I durch $R^1$ dargestellten Alkylrest handelt es sich um Alkyl mit 1 bis 4 C-Atomen; besonders bevorzugte Beispiele sind Methyl und Ethyl.

Bei dem in Formel I durch $R^1$ dargestellten Arylrest handelt es sich bevorzugt um Phenyl, das unsubstituiert ist oder substituiert sein kann, z.B. durch Alkoxy wie Methoxy oder Hexadecyloxy, Benzyloxy oder Phenoxy.

Eine in Formel I angegebene Aminogruppe ist vorzugsweise eine sekundäre Aminogruppe, wie eine Dialkylaminogruppe (Dimethylamino, Diethylamino) oder eine cyclische Aminogruppe wie Piperidino, Pyrrolidino oder Morpholino.

Bevorzugte Kuppler der Erfindung lassen sich durch die nachfolgende allgemeine Formel II beschreiben:

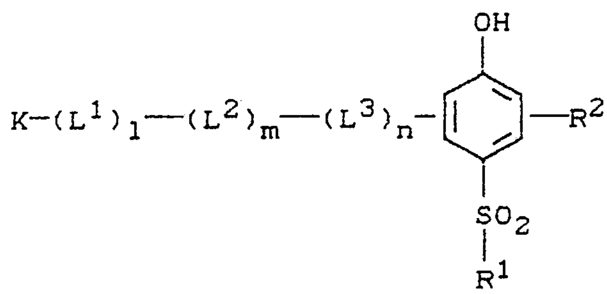

II

worin K, und $R^1$ die angegebene Bedeutung haben und worin bedeuten

$R^2$ Wasserstoff oder einen Rest der Formel

$$-(L^3-)_n-(L^2-)_m-(L^1-)_l-K$$

l, m, n unabhängig voneinander 0 oder 1;

$L^1$ -NH- oder

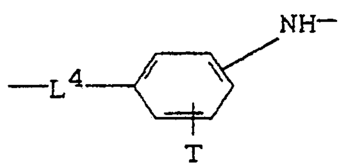

$L^2$ -CO-, $-CO-CH_2-CH_2-CO-$,

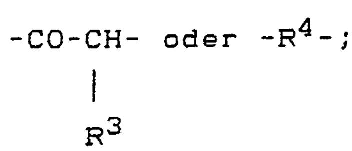

$L^3$ -O-, -NH- oder $-NH-L^5-$;

$L^4$ -O-, -S-, $-NR^5-$ oder $-R^6-$;

$L^5$ $-R^7$ oder

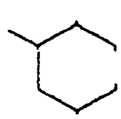

;

T Wasserstoff, Halogen oder Alkoxy;
$R^3$, $R^5$ Wasserstoff oder Alkyl mit 1 bis 20 C-Atomen;
$R^4$, $R^6$, $R^7$ Alkylen mit 2 bis 4 C-Atomen.

Beispielhaft sind im folgenden einige Kuppler der Erfindung angegeben.

Y-1

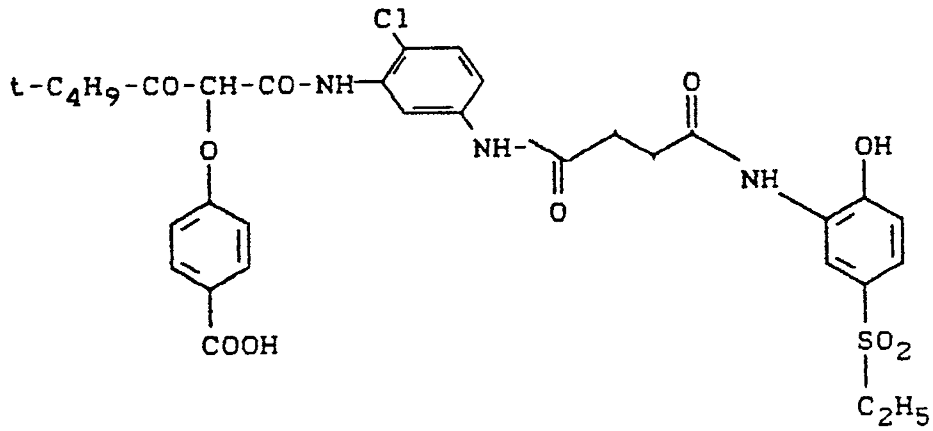

Y-2

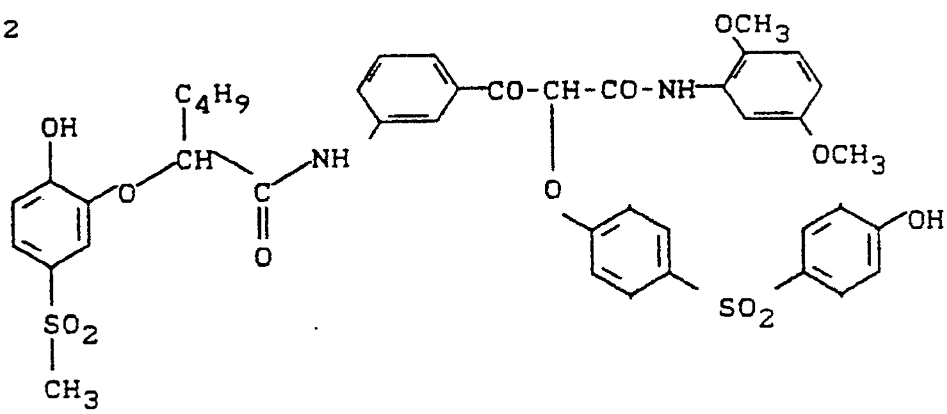

Y-3

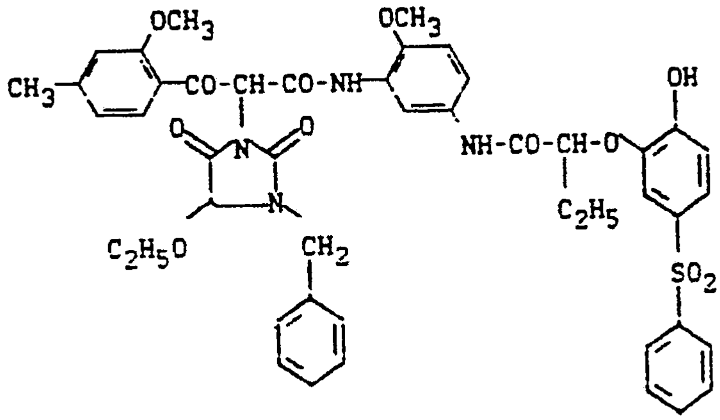

Y-4

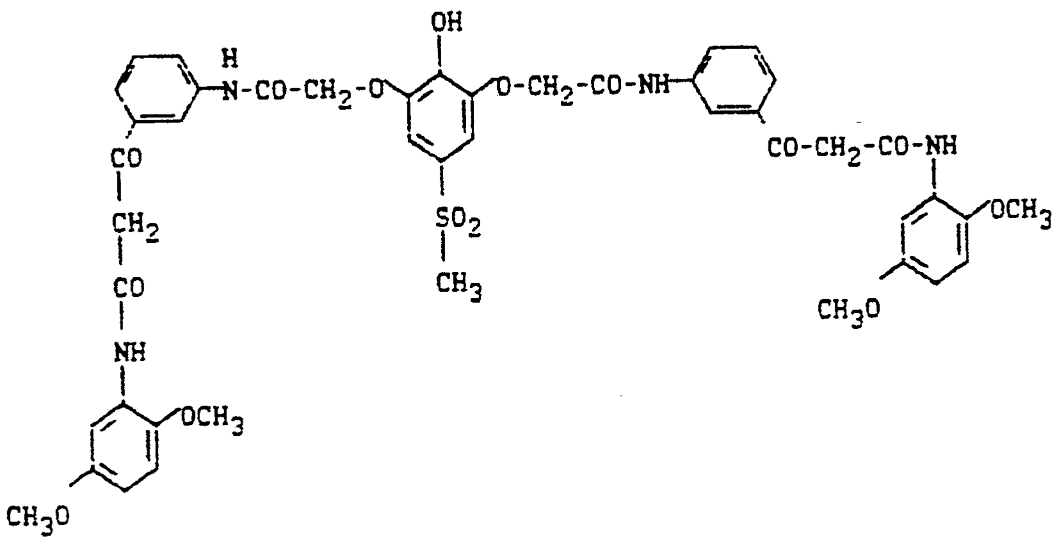

Y-5

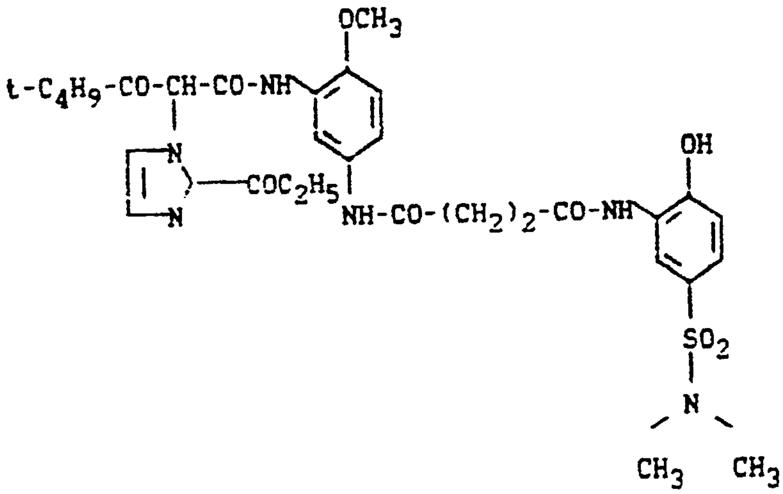

Y-6

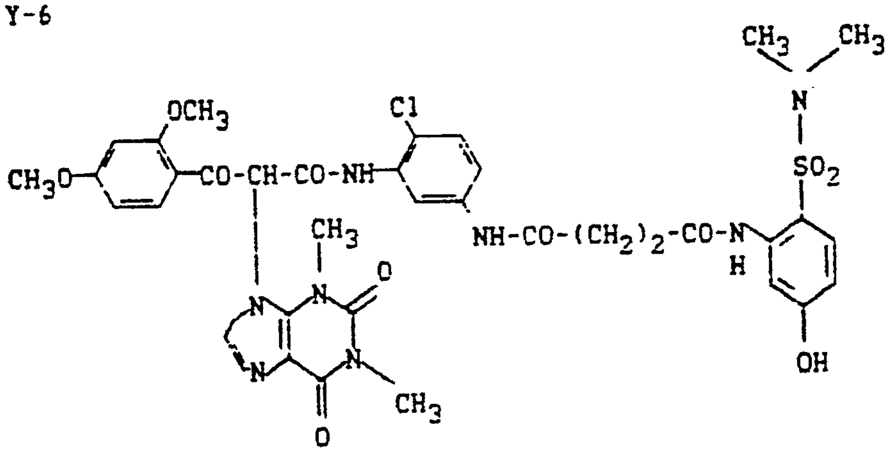

Y-7

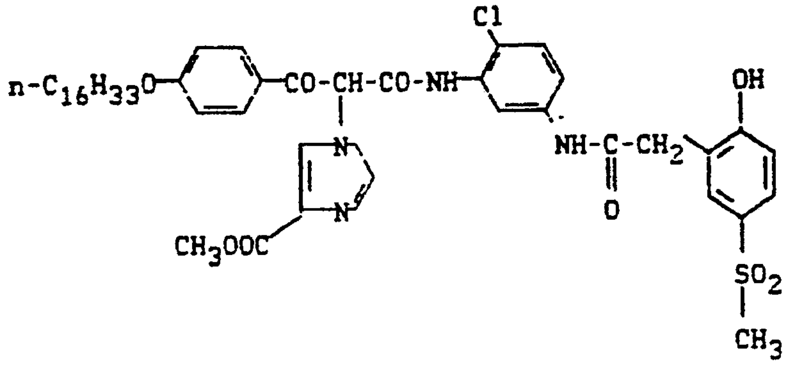

M-1

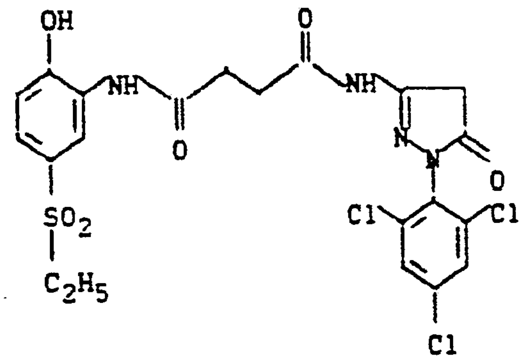

M-2

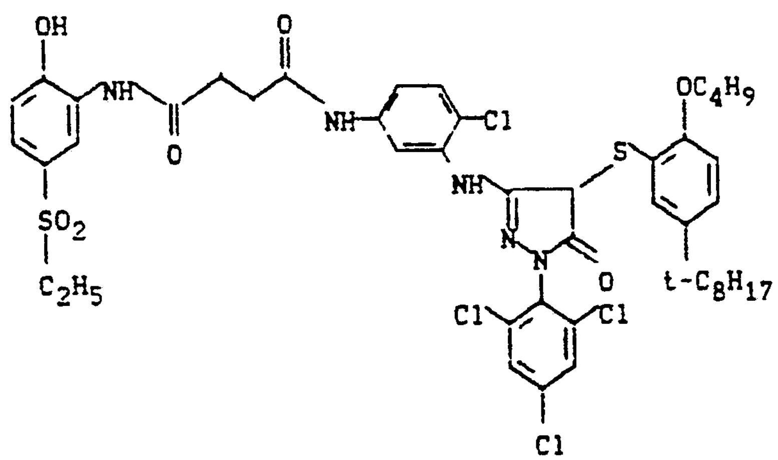

M-3

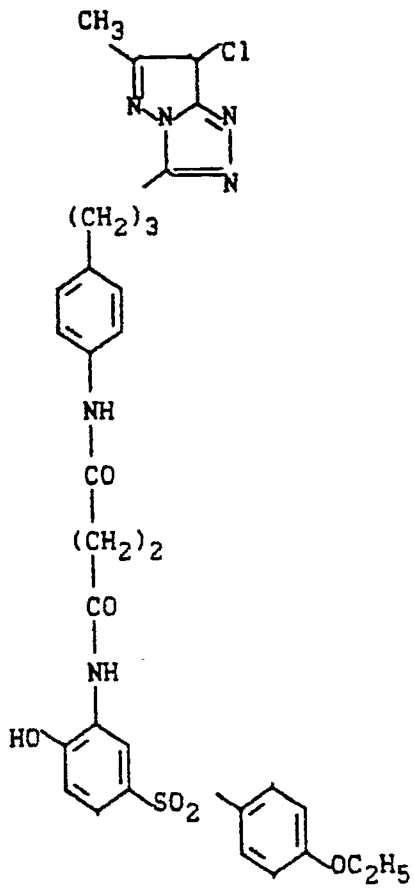

M-4

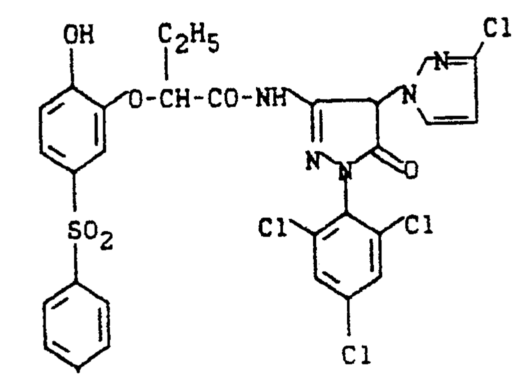

M-5

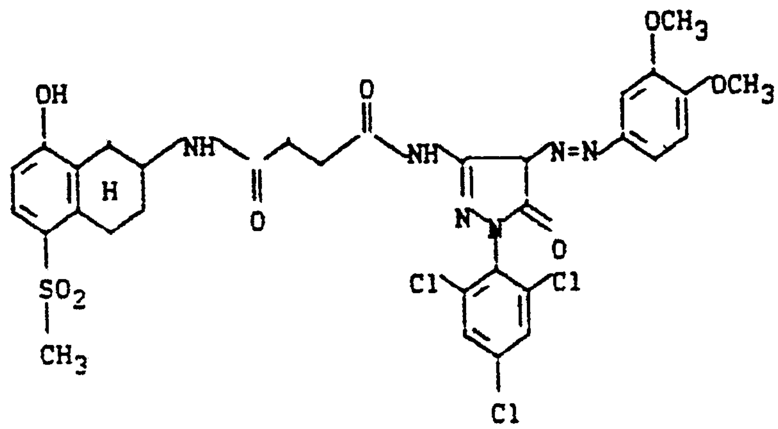

M-6

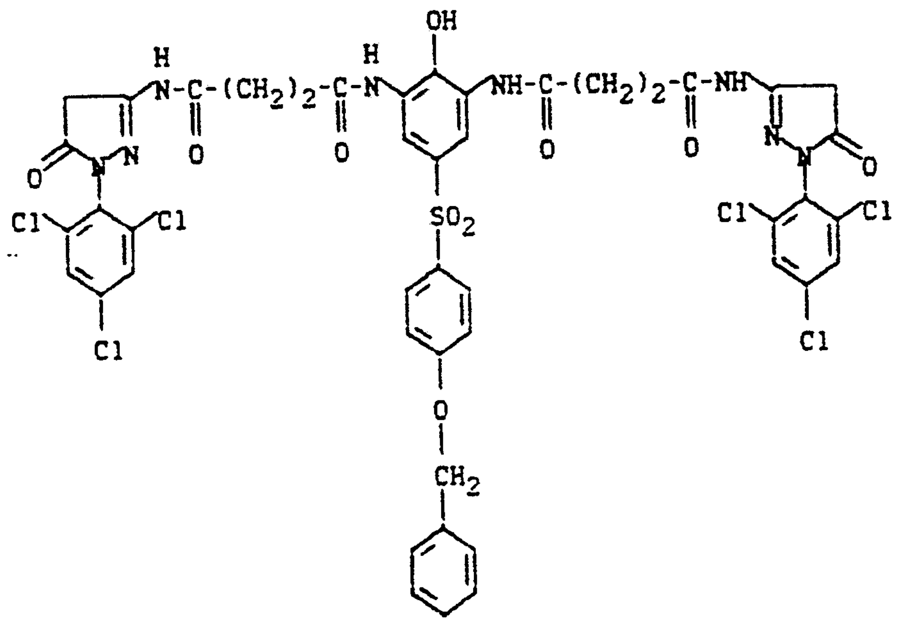

M-7

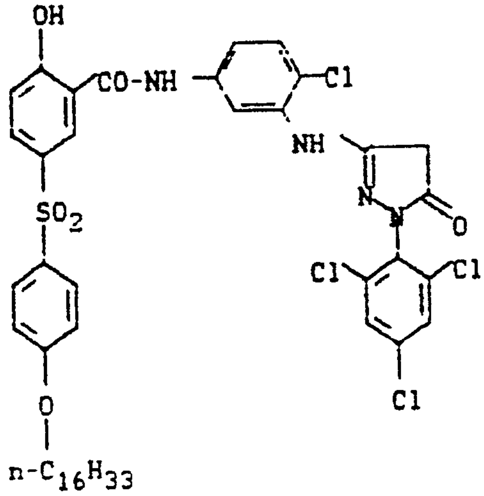

M-8

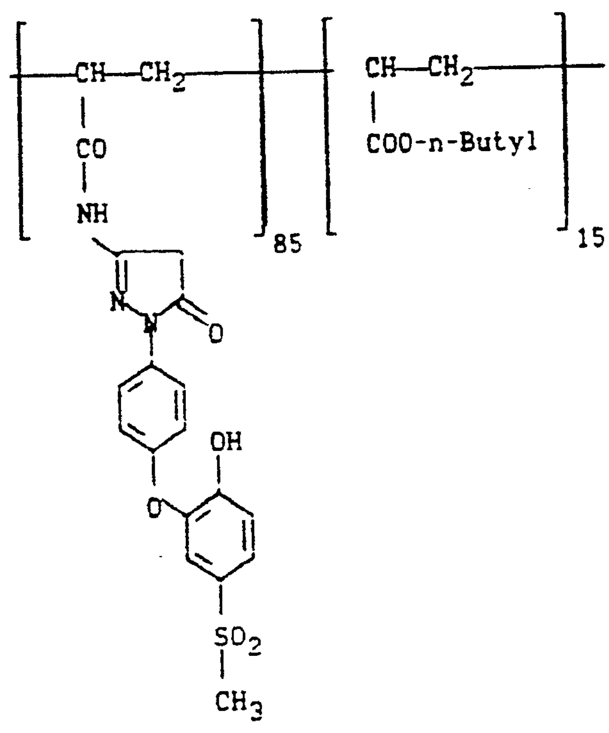

M-9

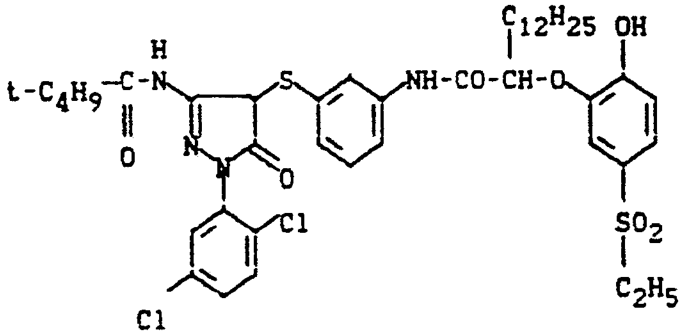

C-1

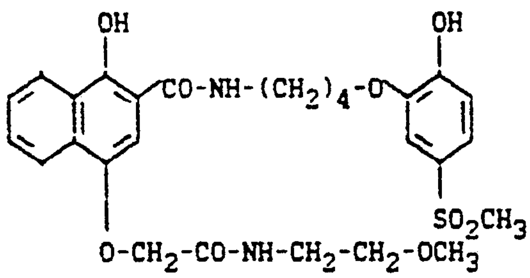

C-2

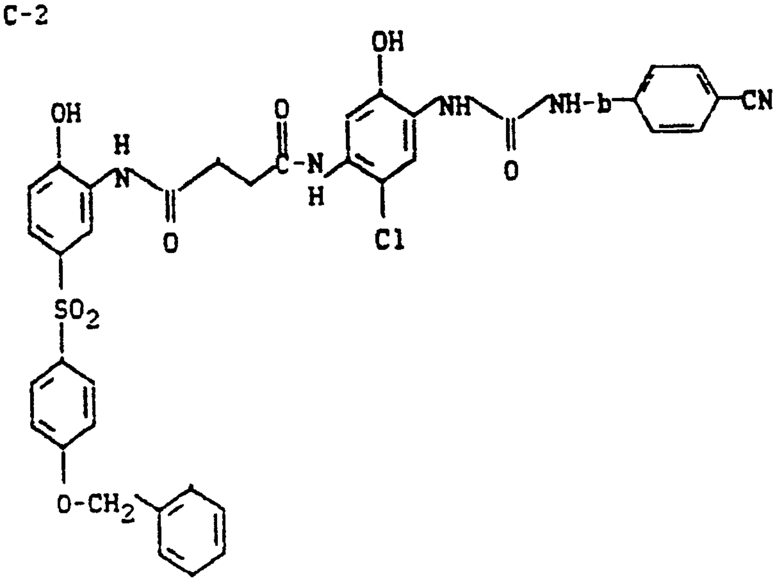

C-3

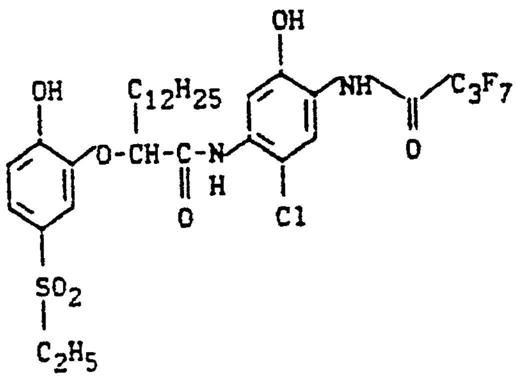

C-4

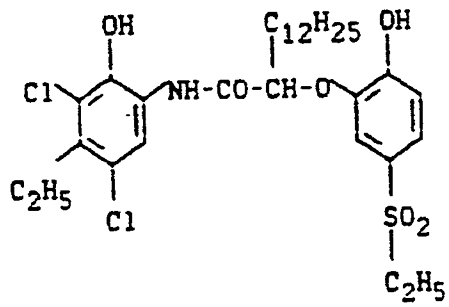

C-5

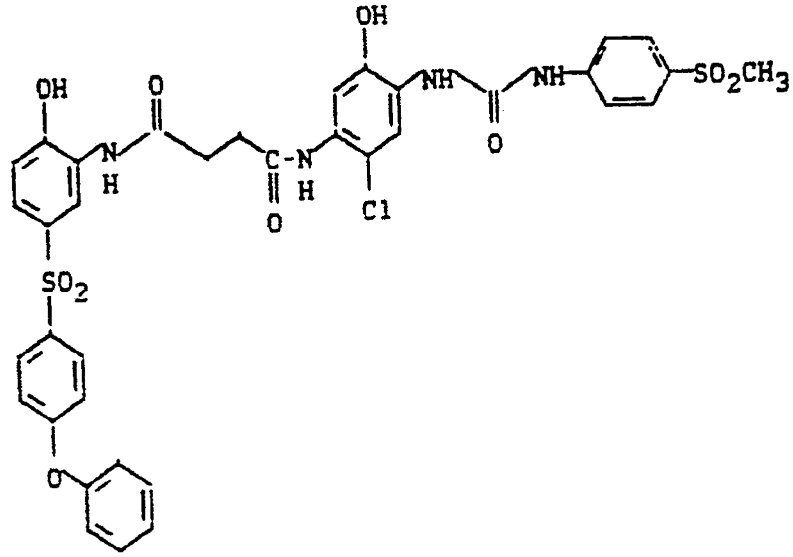

C-6

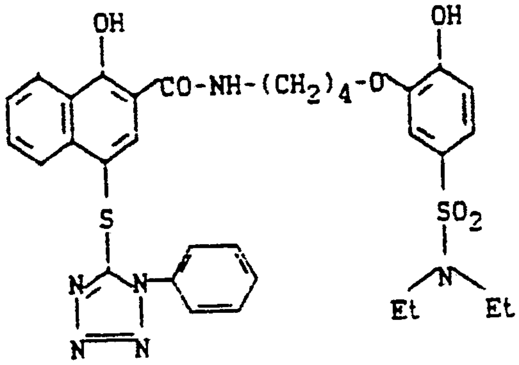

C-7

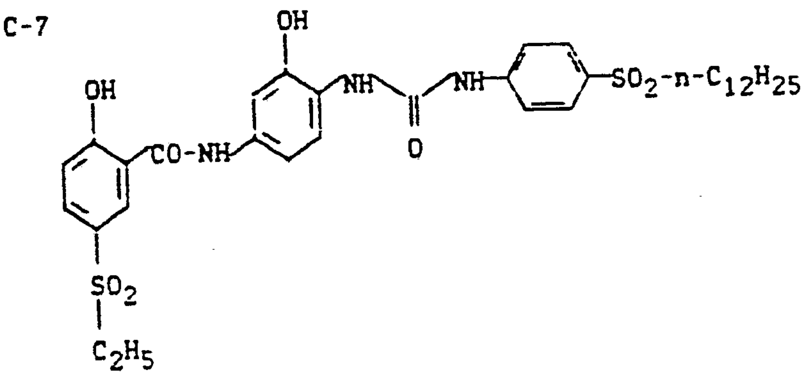

C-8

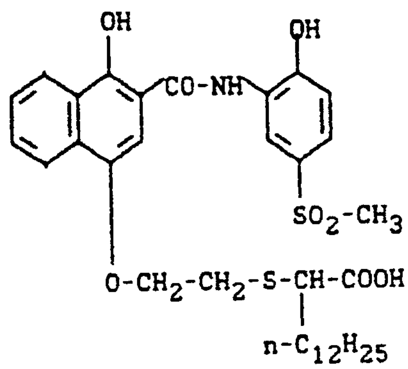

C-9

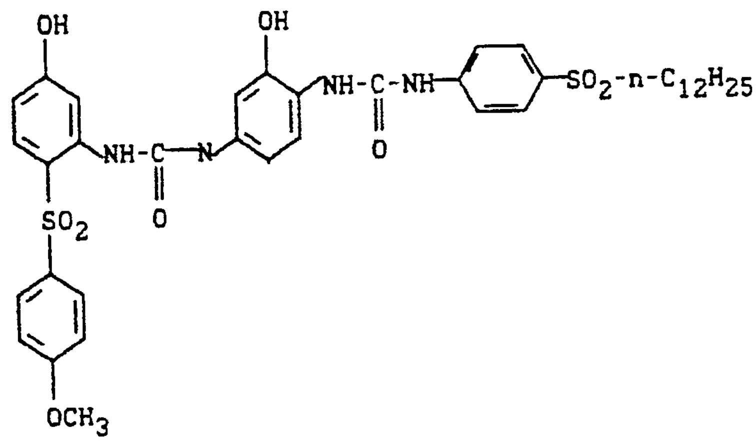

Beispielhaft wird im folgenden die Synthese einiger der erfindungsgemäßen Kuppler beschrieben.

Herstellungsbeispiel 1

Herstellung von Kuppler M-3

29 g 7-Chlor-6-Methyl-3-[3-(p-aminophenyl)-propyl-] 1H-pyrazolo[3,2-c]-s-triazol hergestellt gemäß EP-A-0073636 wurden in

250 ml i-Propanol gelöst und mit 25 ml N,N-Dimethylanilin versetzt. Bei 10°C wurden innerhalb von 30 min 41 g β-[2-Hydroxy-5-(4-ethoxyphenylsulfonyl)-anilino-carbonyl]-propionylchlorid gelöst in 50 ml Dioxan zugetropft. Es wurde 1 h nachgerührt, in 1 l Eis/HCl eingetragen und die wäßrige Phase mit 500 ml Ethylacetat ausgeschüttelt. Nach dem Trocknen über Natriumsulfat wurde das Lösungsmittel unter reduziertem Druck abgezogen und der Rückstand säulenchromatographisch an Kieselgel mit Ethylacetat/Methylchlorid (10:1) getrennt.

Ausbeute: 50 g

Schmp.: 163,5°C Zersetzung.

Herstellungsbeispiel 2

Herstellung von Kuppler C-2

30,3 g 2-(p-Cyanophenylureido)-4-chloro-5-aminophenol hergestellt gemäß EP-A-0028099 wurden in 250 ml Tetrahydrofuran mit 25 ml N,N-Dimethylanilin versetzt. Anschließend tropfte man bei 10°C innerhalb von 30 min 47,2 g β-[2-Hydroxy-5-(4-benzyloxyphenylsulfonyl)-anilinocarbonyl)-propionylchlorid, gelöst in 100 ml Tetrahydrofuran, zu. Es wurde 1 h bei Raumtemperaur nachgerührt, in Eis/HCl gefällt und das

ausgefallene Produkt aus Acetonitril umkristallisiert.
Ausbeute : 45 g
Schmp.: 174 °C

Herstellungsbeispiel 3

Herstellung von Kuppler Y-1

40,5 g α-Pivaloyl-α-(4-carboxy-phenoxy)-2-chloro-5-aminoacetanilid wurden in 250 ml Tetrahydrofuran gelöst und mit 25 ml N,N-Dimethylanilin versetzt. Anschließend tropfte man bei 10 °C innerhalb von 30 min 31,9 g β-[2-Hydroxy-5-ethylsulfonyl-anilinocarbonyl]-propionylchlorid gelöst in 100 ml Tetrahydrofuran zu. Es wurde 1 h bei Raumtemperatur nachgerührt, in 1 l Eis/HCl eingtragen und das ausgefallene Öl nach dem Erstarren abgesaugt. Nach Umkristallisieren aus Dioxan/Aceton (4:1) wurden 65 g Kuppler Y-1 erhalten.
Schmp. 138 - 140 °C.

Die erfindungsgemäßen Kuppler mit dem durch eine Hydroxylgruppe und eine Sulfonylgruppe substituierten Phenylrest weisen genügend hydrophile Eigenschaften auf, um auch ohne besondere Entwicklerzusätze, wie beispielsweise Benzylalkohol, die als Vermittler zwischen hydrophiler und hydrophober Phase anzusehen sind, die Entwicklung der Farbstoffe aus Farbkuppler mit der geforderten Empfindlichkeit und maximalen Farbdichte zu ermöglichen.

Die erfindungsgemäßen Kuppler zeichnen sich weiterhin vor allem durch eine ausgezeichnete Löslichkeit und geringe Kristallisationstendenz in organischen Lösungsmitteln, insbesondere in mit Wasser nicht mischbaren Lösungsmitteln mit hohem Siedepunkt, wie z.B. Trikresylphosphat oder Dibutylphthalat aus.

Dies wirkt sich günstig im Hinblick auf eine geringe Schichtbelastung aus.

Außerdem weisen die Kuppler eine hervorragende Diffusionsfestigkeit in fotografischen Schichten auf, und zwar sowohl beim Gießvorgang als auch während der fotografischen Verarbeitung.

Ein weiterer Vorteil der erfindungsgemäßen Farbkuppler ist ihre hohe Stabilität gegenüber Feuchtigkeit und Wärme, sowie auch die Stabilität der aus ihnen hergestellten Farbstoffe gegenüber Wärme, Feuchtigkeit und Lichteinstrahlung.

Schließlich sind die Farbkuppler der vorliegenden Erfindung gegen Schwankungen des pH bei der Verarbeitung, insbesondere bei der Entwicklung, vergleichsweise unempfindlich.

Die Kombination von ausgezeichneter Löslichkeit in organischen Lösungsmitteln und hervorragender Diffusionsfestigkeit einerseits mit guter Wasserverträglichkeit andererseits ist eine besonders überraschende, unvorhersehrbare Eigenschaft der erfindungsgemäßen Kuppler.

Bei der Herstellung des lichtempfindlichen farbfotografischen Aufzeichnungsmaterials können die diffusionsfesten Kuppler der vorliegenden Erfindung in bekannter Weise in die Gießlösung der Silberhalogenidemulsionsschichten oder anderer Kolloidschichten eingearbeitet werden. Beispielsweise können die öllöslichen oder hydrophoben Kuppler vorzugsweise aus einer Lösung in einem geeigneten Kupplerlösngsmittel (Ölbildner) gegebenenfalls in Anwesenheit eines Netz- oder Dispergiermittels zu einer hydrophilen Kolloidlösung zugefügt werden. Die hydrophile Gießlösung kann selbstverständlich neben dem Bindemittel andere übliche Zusätze enthalten. Die Lösung des Kupplers braucht nicht direkt in die Gießlösung für die Silberhalogenidemulsionsschicht oder eine andere wasserdurchlässige Schicht dispergiert zu werden; sie kann vielmehr auch vorteilhaft zuerst in einer wäßrigen nichtlichtempfindlichen Lösung eines hydrophilen Kolloids dispergiert werden, worauf das erhaltene Gemisch gegebenenfalls nach Entfernung der verwendeten niedrig siedenden organischen Lösungsmittel mit der Gießlösung für die lichtempfindliche Silberhalogenidemulsionsschicht oder einer anderen wasserdurchlässigen Schicht vor dem Auftragen vermischt wird.

Als lichtempfindliche Silberhalogenidemulsionen eignen sich Emulsionen von Silberchlorid, Silberbromid oder Gemischen davon, evtl. mit einem geringen Gehalt an Silberiodid bis zu 10 mol-% in einem der üblicherweise verwendeten hydrophilen Bindmittel. Als Bindemittel für die fotografischen Schichten wird vorzugsweise Gelatine verwendet. Diese kann jedoch ganz oder teilweise durch andere natürliche oder synthetische Bindemittel ersetzt werden.

Die Emulsionen können in der üblichen Weise chemisch oder spektral sensibilisiert sein und die Emulsionsschichten wie auch andere nicht-lichtempfindliche Schichten können in der üblichen Weise mit bekannten Härtungsmitteln gehärtet sein.

Üblicherweise enthalten farbfotografische Aufzeichnungsmaterialen mindestens je eine Silberhalogenidemulsionsschicht für die Aufzeichnung von Licht jedes der drei Spektralbereiche Rot, Grün und Blau. Zu diesem Zweck sind die lichtempfindlichen Schichten in bekannter Weise durch geeignete Sensibilisierungsfarbstoffe spektral sensibilisiert. Blauempfindliche Silberhalogenidemulsionsschichten müssen nicht notwendigerweise einen Spektralsensibilisator enthalten, da für die Aufzeichnung von blauem Licht in vielen Fällen die Eigenempfindlichkeit des Silberhalogenids ausreicht.

Jede der genannten lichtempfindlichen Schichten kann aus einer einzigen Schicht bestehen oder in bekannter Weise, z.B. bei der sogenannten Doppelschichtanordnung, auch zwei oder auch mehr Silberhalogenidemulsionsteilschichten umfassen (DE-C-1 121 470). Üblicherweise sind rotempfindliche Silberhalogenidemulsionsschichten dem Schichtträger näher angeordnet als grünempfindliche Silberhalogenidemulsionsschichten und diese wiederum näher als blauempfindliche, wobei sich im allgemeinen zwischen grünempfindlichen Schichten und blauempfindlichen Schichten eine nicht lichtemfindliche gelbe Filterschicht befindet. Es sind aber auch andere Anordnungen denkbar. Zwischen Schichten unterschiedlicher Spektralempfindlichkeit ist in der Regel eine nicht lichtempfindliche Zwischenschicht angeordnet, die Mittel zur Unterbindung der Fehldiffusion von Entwickleroxidationsprodukten enthalten kann. Falls mehrere Silberhalogenidemulsionsschichten gleicher Spektralempfindlichkeit vorhanden sind, können diese einander unmittelbar benachbart sein oder so angeordnet sein, daß sich zwischen ihnen eine lichtempfindliche Schicht mit anderer Spektralempfindlichkeit befindet (DE-A-1 958 709, DE-A-2 530 645, DE-A-2 622 922).

Farbfotografische Aufzeichnungsmaterialien zur Herstellung mehrfarbiger Bilder enthalten üblicherweise in räumlicher und spektraler Zuordnung zu den Silberhalogenidemulsionsschichten unterschiedlicher Spektralempfindlichkeit farbgebende Verbindungen, hier besonders Farbkuppler, zur Erzeugung der unterschiedlichen Teilfarbenbilder Cyan, Purpur und Gelb.

Unter räumlicher Zuordnung ist dabei zu verstehen, daß der Farbkuppler sich in einer solchen räumlichen Beziehung zu der Silberhalogenidemulsionsschicht befindet, daß eine Wechselwirkung zwischen ihnen möglich ist, die eine bildgemäße Übereinstimmung zwischen dem bei der Entwicklung gebildeten Silberbild und dem aus dem Farbkuppler erzeugten Farbbild zuläßt. Dies wird in der Regel dadurch erreicht, daß der Farbkuppler in der Silberhalogenidemulsionsschicht selbst enthalten ist oder in einer hierzu benachbarten gegebenenfalls nichtlichtempfindlichen Bindemittelschicht.

Unter spektraler Zuordnung ist zu verstehen, daß die Spektralempfindlichkeit jeder der lichtempfindlichen Silberhalogendemulsionsschichten und die Farbe des aus dem jeweils räumlich zugeordneten Farbkuppler erzeugten Teilfarbenbildes in einer bestimmten Beziehung zueinander stehen, wobei jeder der Spektralempfindlichkeiten (Rot, Grün, Blau) eine andere Farbe betreffenden Teilfarbenbildes (im allgemeinen z.B. die Farben Cyan, Purpur bzw. Gelb in dieser Reihenfolge) zugeordnet ist.

Jeder der unterschiedlich spektral sensibilisierten Silberhalogenidemulsionsschichten kann ein oder können auch mehrere Farbkuppler zugeordnet sein. Wenn mehrere Silberhalogenidemulsionsschichten gleicher Spektralempfindlichkeit vorhanden sind, kann jede von ihnen einen Farbkuppler enthalten, wobei diese Farbkuppler nicht notwendigerweise identisch zu sein brauchen. Sie sollen lediglich bei der Farbentwicklung wenigstens annähernd die gleiche Farbe ergeben, normalerweise eine Farbe, die komplementär ist zu der Farbe des Lichtes, für das die betreffenden Silberhalogenidemulsionsschichten überwiegend empfindlich sind.

Rotempfindlichen Silberhalogenidemulsionsschichten ist folglich bei bevorzugten Ausführungsformen mindestens ein nichtdifundierender Farbkuppler zur Erzeugung des blaugrünen Teilfarbenbildes zugeordnet, in der Regel ein Kuppler vom Phenol- oder $\alpha$-Naphtholtyp. Grünempfindlichen Silberhalogenidemulsionsschichten ist mindestens ein nichtdiffundierender Farbkuppler zur Erzeugung des purpurnen Teilfarbenbildes zugeordnet, wobei üblicherweise Farbkuppler vom Typ des 5-Pyrazolons, des Indazolons oder des Pyrazolotriazols Verwendung finden. Blauempfindlichen Silberhalogenidemulsionsschichten schließlich ist mindestens ein nichtdiffundierender Farbkuppler zur Erzeugung des gelben Teilfarbenbildes zugeordnet, in der Regel ein Farbkuppler mit einer offenkettigen Ketomethylengruppierung. Farbkuppler dieser Art sind in großer Zahl bekannt und in einer Vielzahl von Patentschriften beschrieben. Beispielhaft sei hier auf die Veröffentlichungen "Farbkuppler" von W. PELZ in "Mitteilungen aus den Forschungslaboratorien der Agfa, Leverkusen/München", Band III, Seite 111 (1961) und von K. VENKATARAMAN in "The Chemistry of Synthetic Dyes", Vol. 4, 341 bis 387, Academic Press (1971), verwiesen.

Bei den Farbkupplern kann es sich sowohl um übliche 4-Äquivalentkuppler handeln als auch um 2-Äquivalentkuppler, bei denen zur Farberzeugung eine geringere Menge Silberhalogenid erforderlich ist. 2-Äquivalentkuppler leiten sich bekanntlich von den 4-Äquivalentkupplern dadurch ab, daß sie in der Kupplungsstelle einen Substituenten enthalten, der bei der Kupplung abgespalten wird. Zu den 2-Äquivalentkupplern sind sowohl solche zu rechnen, die praktisch farblos sind, als auch solche, die eine intensive Eigenfarbe aufweisen, die bei der Farbkupplung verschwindet bzw. durch die Farbe des erzeugten Bildfarbstoffes ersetzt wird. Letztere Kuppler können ebenfalls zusätzlich in den lichtempfindlichen Silberhalogenidemulsionsschichten vorhanden sein und dort als Maskenkuppler zur Kompensierung der unerwünschten Nebendichten der Bildfarbstoffe dienen. Zu den 2-Äquivalenkupplern sind aber such die bekannten Weißkuppler zu rechnen, die jedoch bei Reaktion mit Farbentwickleroxidationsprodukten keinen Farbstoff ergeben. Zu den 2-Äquivalentkupplern sind ferner die bekannten DIR-Kuppler zu rechnen, bei denen es sich um Kuppler handelt, die in der Kupplungsstelle einen abspaltbaren Rest enthalten, der bei

Reaktion mit Farbentwickleroxidationsprodukten als diffundierender Entwicklungsinhibitor in Freiheit gesetzt wird. Auch andere fotografisch wirksame Verbindungen, z.B. Entwicklungsaceferatoren oder Schleiermittel, können bei der Entwicklung aus solchen Kupplern freigesetzt werden.

Erfindungsgemäß enthält das farbfotografische Aufzeichnungsmaterial mindestens einen Kuppler, bevorzugt mindestens einen Farbkuppler, der in Formel I angegebenen Struktur. Die hierdurch erzielten Vorteile sind beispielsweise aus den nachfolgend beschriebenen Beispielen ersichtlich. Obwohl die genauen Zusammenhänge nicht im einzelnen bekannt sind, wird angenommen, daß die mit den erfindungsgemäßen Kupplern erzielten Vorteile auf der Struktur der in Formel I dargestellten Kuppler, insbesondere der speziellen Struktur des durch eine Hydroxylgruppe und eine Sulfonylgruppe substituierten Phenylrestes beruht.

Die charakteristische Gruppe der Kuppler der Formel I ist nicht Bestandteil des Kupplerrests K; sie hat keinen wesentlichen Einfluß auf die spektralen Eigenschaften der erzeugten Bildfarbstoffe. Vielmehr wirkt sie sich als sogenannter Emulgierrest förderlich auf die Dispergierbarkeit der Kuppler und Stabilität der farbfotografischen Aufzeichnungsmaterialien aus. Es ist somit auch möglich, daß das farbfotografische Aufzeichnungsmaterial mehrere verschiedene Kuppler der Formel I enthält, wobei beispielsweise mehreren oder jeder der unterschiedlich spektral sensibilisierten Silberhalogendemulsionsschichten ein solcher Kuppler zugeordnet sein kann.

Über die genannten Bestandteile hinaus kann das farbfotografische Aufzeichnungsmaterial der vorliegenden Erfindung weitere Zusätze enthalten, wie zum Beispiel Antioxidantien, farbstoffstabilisierende Mittel und Mittel zur Beeinflussung der mechanischen und elektrostatischen Eigenschaften. Um die nachteilige Einwirkung von UV-Licht auf die mit dem erfindungsgemäßen farbfotografischen Aufzeichnungsmaterial hergestellten Farbbilder zu vermindern oder zu vermeiden, ist es beispielsweise vorteilhaft, in einer oder mehreren der in dem Aufzeichnungsmaterial enthaltenen Schichten, vorzugsweise in einer der oberen Schichten, UV-absorbierende Verbindungen zu verwenden. Geeignete UV-Absorber sind beispielsweise in US-A-3 253 921, DE-C-2 036 719 und EP-A-0 057 160 beschrieben.

Zur Herstellung farbfotografischer Bilder wird das erfindungsgemäße farbfotografische Aufzeichnungsmaterial, das mindestens eine Silberhalogenidemulsionsschicht und mindestens einen dieser zugeordneten Kuppler der Formel I enthält, mit einer Farbentwicklerverbindung entwickelt. Als Farbentwicklerverbindung lassen sich sämtliche Entwicklerverbindungen verwenden, die die Fähigkeit besitzen in Form ihres Oxidationsproduktes mit Farbkupplern zu Azomethinfarbstoffen zu reagieren. Geeignete Farbentwicklerverbindungen sind aromatische mindestens eine primäre Aminogruppe enthaltende Verbindungen vom p-Phenylendiamintyp, beispielsweise N,N-Dialkyl-p-phenylendiamine, wie N,N-Diethyl-p-phenylendiamin, 1-(N-ethyl-N-methylsulfonamidoethyl)-3-methyl-p-phenylendiamin, 1-(N-ethyl-N-hydroxyethyl-3-methyl-p-phenylendiamin und 1-(N-ethyl-N-methoxyethyl)-3-methyl-p-phenylendiamin.

Beispiel 1

Je 0,03 mol der in der nachstehenden Tabelle 1 aufgeführten Kuppler wurden unter Erwärmen auf 60 °C in der gleichen Gewichtsmenge Dibutylphthalat und der dreifachen Gewichtsmenge Ethylacetat gelöst. Die Lösungen wurden anschließend unter Zusatz von Alkanol B, einem Alkylnaphthalinsulfonat, hergestellt von DuPont, in wäßriger Gelatine dispergiert. Die Dispergate wurden mit einer Silberchlorbromidemulsion (20 mol-% AgBr) aus 0,1 mol Silber vermischt, auf einen polyethylen-kaschierten Papierträger aufgetragen und getrocknet. Die so hergestellten fotografischen Materialien (Proben 1 bis 7) wurden hinter einem Graukeil belichtet und wie folgt einmal mit und einmal ohne Benzylalkohol verarbeitet (30 °C):

| | |
|---|---|
| Entwickeln | 210 s |
| Bleichfixieren | 90 s |
| Wässern | 120 s |

Die Bäder hatten folgende Zusammensetzung:

| | |
|---|---|
| 5,0 g | 4-Amino-3-methyl-N-ethyl-N-(ß-methansulfonamidoethyl)-anilin-sulfat |
| (15,0 ml | Benzylalkohol) |
| 2,5 g | Natriumhexametaphosphat |
| 1,85 g | $Na_2SO_3$ sicc. |
| 1,4 g | NaBr |
| 0,5 g | KBr |
| 39,1 g | Borax |

auffüllen mit Wasser auf 1000 ml; mit NaOH auf pH 10,3 einstellen.

Bleichfixierbad:

| | |
|---|---|
| 50,0 g | Ethylendiamintetraessigsäure-Eisen(III)-Ammonium-Komplex |
| 50,0 ml | $(NH_4)_2SO_3$, 40%ige Lösung |
| 140,0 ml | $(NH_4)_2SSO_3$, 70%ige Lösung |
| 20,0 ml | wäßrige Ammoniak, 28%ig |
| 4,0 g | Ethylendiamin tetraessigsäure |

auffüllen mit Wasser auf 1000 ml.

Für jede der hergestellten Proben 1 bis 7 sind in der Tabelle 1 die relative Empfindlichkeit und die maximale Farbdichte angegeben. Für die Empfindlichkeit werden relative Werte angegeben, bezogen auf die höchste erzielte Empfindlichkeit (=100). Die bei Entwicklung in Gegenwart von Benzylalkohol erzielten Werte sind in Klammern aufgeführt.

Tabelle 1

| Probe | Kuppler | rel. Empf. | | Dmax | |
|---|---|---|---|---|---|
| 1 | Y-1 | 85 | (99) | 2,35 | (2,56) |
| 2 | M-3 | 92 | (100) | 2,14 | (2,82) |
| 3 | M-7 | 90 | (97) | 2,32 | (2,75) |
| 4 | C-3 | 88 | (96) | 2,01 | (2,58) |
| 5 | V-1 | 70 | (89) | 1,48 | (1,99) |
| 6 | V-2 | 55 | (92) | 1,50 | (2,01) |
| 7 | V-3 | 61 | (91) | 1,32 | (1,98) |

Folgende Vergleichskuppler wurden verwendet:

V-1 (= Y-7 aus US-A-4 503 141)

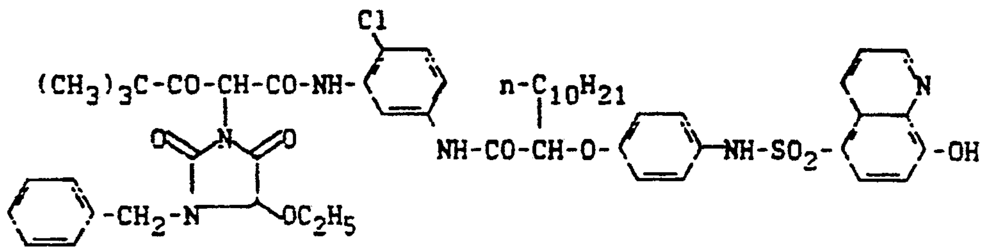

V-2 (= M-11 aus US-A-4 513 082)

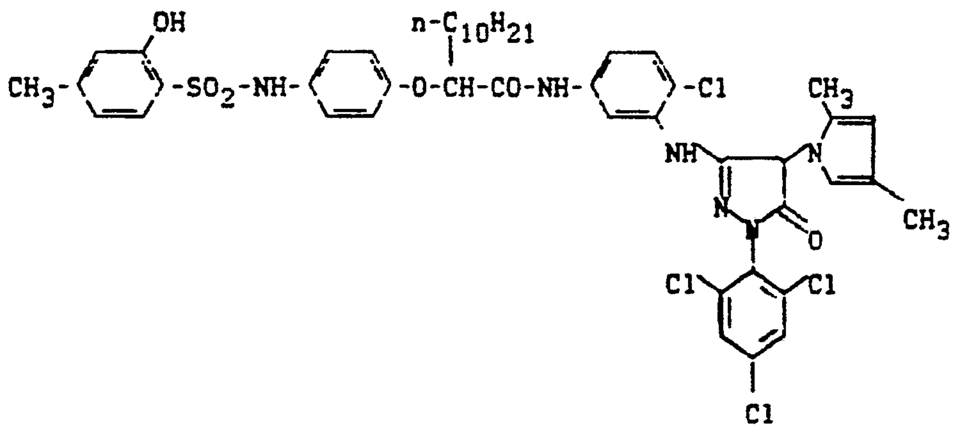

V-3 (= C-3 aus US-A-4 503 141)

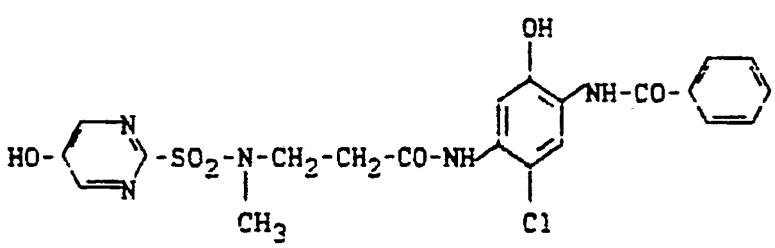

Man erkannt aus Tabelle 1, daß die erfindungsgemäßen Kuppler hohe Empfindlichkeit und hohe Farbdichte liefern, selbst bei Entwicklung in Abwesenheit von Benzylalkohol. Sie sind daher den Vergleichskupplern überlegen.

Beispiel 2

In der gleichen Weise wie in Beispiel 1 beschrieben, wurden Proben 8 bis 13 hergestellt und verarbeitet (Entwicklung in Gegenwart von Benzylalkohol). Ebenso wurde in einer weiteren Versuchsreihe verfahren, wobei jedoch dem Bleichfixierbad zusätzlich 5 g Natriumdithionit zugesetzt worden waren, um künstlich ein "erschöpftes" Bleichfixierbad zu erzeugen und seine Wirkung auf die erzielte bg-Farbdichte zu untersuchen. In Tabelle 2 ist die in Reflektion gemessene bg-Dichte (Dmax) angegeben, die mit frischem (DA) bzw. "erschöpftem" (DB) Bleichfixierbad erhalten wird. Die prozentuale Restdichte ist in der letzten Spalte angegeben, wobei die prozentuale Restdichte nach folgender Gleichung ermittelt wird:

prozentuale Restdichte =

$$\frac{D_B}{D_A} \times 100$$

Tabelle 2

| Probe | Kuppler | Farbdichte $D_{max}$ frisches / Bleichfixierbad | erschöpftes" Bleichfixierbad | Restdichte [%] |
|---|---|---|---|---|
| 8 | C-5 | 2,35 | 2,25 | 96 |
| 9 | C-7 | 2,42 | 2,28 | 94 |
| 10 | C-9 | 2,28 | 2,20 | 96,5 |
| 11 | V-3 | 2,05 | 1,84 | 90 |
| 12 | V-4 | 1,98 | 1,35 | 68 |
| 13 | V-5 | 1,95 | 1,55 | 79,5 |

Aus der Tabelle 2 ist ersichtlich, daß die aus den erfindungsgemäßen Kupplern erzeugten Bildfarbstoffe in geringem Maße einen Farbverlust erleiden, wenn sie in erschöpften Bleichfixierbädern behandelt werden. Dies stellt einen bedeutenden Vorteil gegenüber den bekannten Kupplern dar.

Folgende Vergleichskuppler wurden verwendet (V-3 wurde bereits in Beispiel 1 erwähnt):

V-4

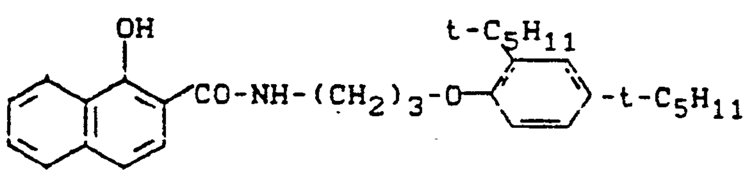

V-5

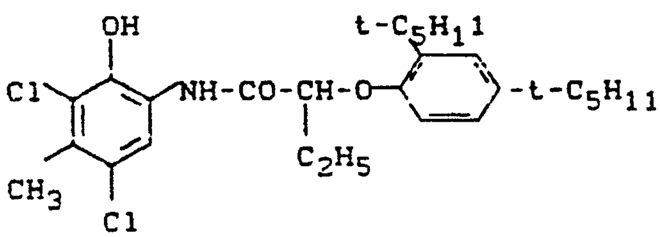

## Ansprüche

1. Farbfotografisches Aufzeichnungsmaterial mit mindestens einer Silberhalogenidemulsionsschicht und mindestens einem Kuppler, der einen mit einer Hydroxylgruppe und einer Sulfonylgruppe substituierten Phenylrest enthält,
dadurch gekennzeichnet, daß der Kuppler der folgenden allgemeinen Formel I entspricht

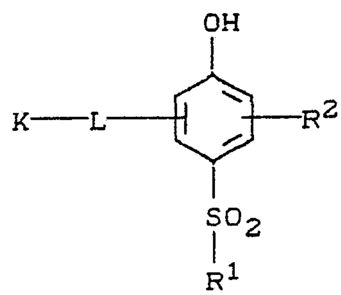

I

worin bedeuten
K einen Kupplerrest
L eine direkte Bindung oder ein Bindeglied
$R^1$ Alkyl mit 1 bis 4 C-Atomen, Aralkyl, Aryl oder Amino
$R^2$ Wasserstoff oder einen Rest -L-K.

2. Aufzeichnungsmaterial nach Anspruch 1, gekennzeichnet durch den Gehalt an einem Kuppler der allgemeinen Formel II

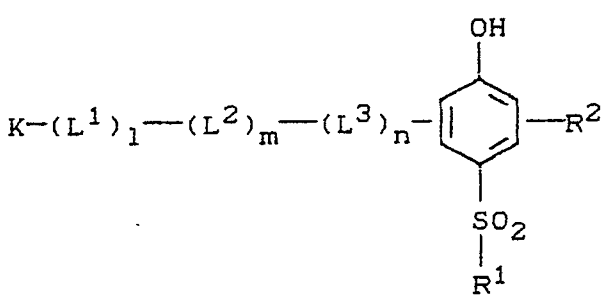

II

worin bedeuten
K einen Kupplerrest
$R^1$ Alkyl mit 1 bis 4 C-Atomen, Aralkyl, Aryl oder Amino
$R^2$ Wasserstoff oder einen Rest der Formel

$$-(L^3-)_n-(L^2-)_m-(L^1-)_l-K$$

$L^1$ -NH- oder

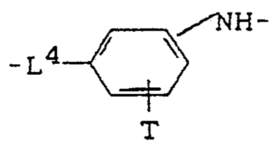

;

$L^2$ -CO-, -CO-$CH_2$ -$CH_2$-CO-,

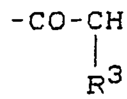

oder $-R^4-$;
$L^3$ -O-, -NH- oder $-NH-L^5-$;
$L^4$ -O-, -S-, $-NR^5-$ oder $-R^6-$;
$L^5$ $-R^7-$ oder

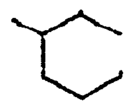

T Wasserstoff, Halogen oder Alkoxy;
$R^3$,$R^5$ Wasserstoff oder Alkyl mit 1 bis 20 C-Atomen;
$R^4$,$R^6$,$R^7$ Alkylen mit 2 bis 4 C-Atomen;
l,m,n unabhängig voneinander 0 oder 1.

**3.** Aufzeichnungsmaterial nach Anspruch 1 oder 2, dadurch gekennzeichnet, daß in den Formeln I oder II K den Rest eines Gelbkupplers, eines Purpurkupplers oder eines Blaugrünkupplers bedeutet.

## Claims

**1.** Colour photographic recording material having at least one silver halide emulsion layer and at least one coupler containing a phenyl group substituted with a hydroxyl group and with a sulphonyl group, characterised in that the coupler corresponds to the following general formula I

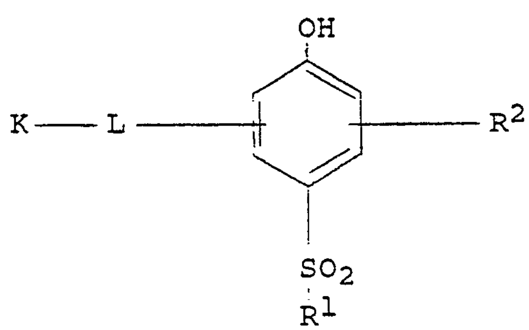

I

wherein

K denotes a coupler group,
L denotes a direct bond or a linking member,
$R^1$ denotes alkyl having 1 to 4 carbon atoms, aralkyl, aryl or amino and
$R^2$ denotes hydrogen or a group -L-K.

**2.** Recording material according to Claim 1, characterised by containing a coupler corresponding to the general formula II

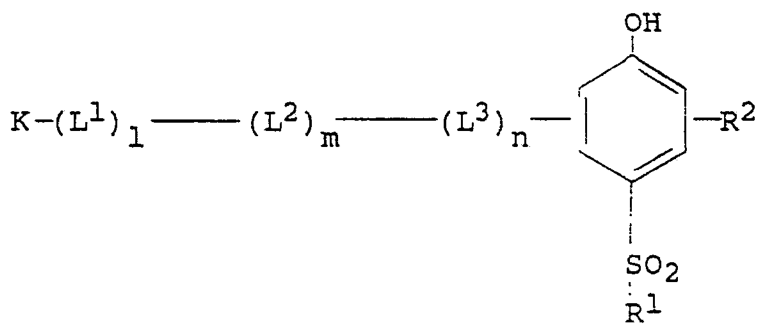 II

wherein
K denotes a coupler group,
$R^1$ denotes alkyl having 1 to 4 carbon atoms, aralkyl, aryl or amino,
$R^2$ denotes hydrogen or a group of the following formula

$$-(L^3-)_n-(L^2-)_m-(L^1-)_l-K$$

wherein
$L^1$ = NH or

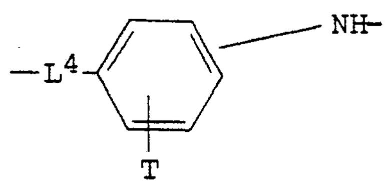 ;

$L^2$ = CO, CO-$CH_2$-$CH_2$-CO,

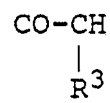

or $R^4$,
$L^3$ = O, NH or NH-$L^5$,
$L^4$ = O, S, $NR^5$ or $R^6$ and
$L^5$ = $R^7$ or

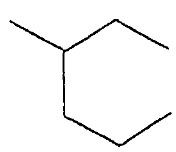 ,

T denotes hydrogen, halogen or alkoxy,
$R^3$ and $R^5$ denote hydrogen or alkyl having 1 to 20 carbon atoms,
$R^4$,$R^6$ and $R^7$ denote alkylene having 2 to 4 carbon atoms, and
l, m, and n denote, independently of one another, 0 or 1.

**3.** Recording material according to Claim 1 or 2, characterised in that K in the formula I or II denotes the residue of a yellow coupler, a magenta coupler or a cyan coupler.

## Revendications

1. Matériau d'enregistrement photographique en couleurs avec au moins une couche d'émulsion d'halogénure d'argent et au moins un coupleur contenant un reste phényle substitué par un groupe hydroxyle et un groupe sulfonyle, caractérisé en ce que le coupleur répond à la formule générale I

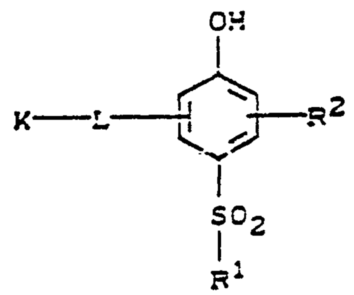

I

dans laquelle

K représente un reste de coupleur

L représente une liaison directe ou un chaînon de liaison

$R^1$ représente un groupe alkyle avec 1 à 4 atomes de carbone, un reste aralkyle, aryle ou amino

$R^2$ représente l'hydrogène ou un reste -L-K.

2. Matériau d'enregistrement selon la revendication 1, caractérisé en ce qu'il contient un coupleur de formule générale II

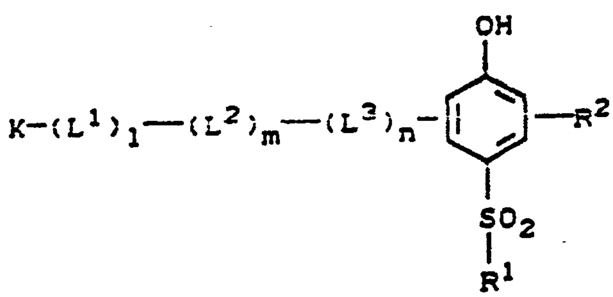

II

dans laquelle

K représente un reste de coupleur

$R^1$ représente un groupe alkyle avec 1 à 4 atomes de carbone, un reste aralkyle, aryle ou amino

$R^2$ représente l'hydrogène ou un reste de formule

$$-(L^3-)_n-(L^2-)_m-(L^1-)_l-K$$

$L^1$ représente -NH- ou

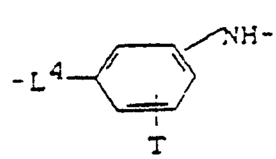

$L^2$ représente -CO-, $-CO-CH_2-CH_2-CO-$,

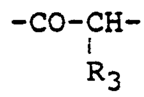

ou $-R^4-$;
$L^3$ représente -O-, -NH- ou $-NH-L^5-$;
$L^4$ -O-, -S-, $-NR^5-$ o u $-R^6$;
$L^5$ représente $-R^7$ ou

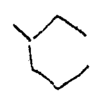

T représente l'hydrogène, un halogène ou un groupe alcoxy;
$R^3$, $R^5$ représentent l'hydrogène ou un groupe alkyle avec 1 à 20 atomes de carbone;
$R^4$, $R^6$, $R^7$ représentent des groupes alkylène avec 2 à 4 atomes de carbone.
l, m, n signifient indépendamment l'un de l'autre 0 ou 1;

3. Matériau d'enregistrement selon la revendication 1 ou 2, caractérisé en ce que dans la formule I ou II, K représente un reste d'un coupleur en jaune, d'un coupleur en pourpre ou d'un coupleur en bleu-vert.